# EUROPÄISCHE PATENTANMELDUNG

(11) **EP 1 933 300 A1**
(43) Veröffentlichungstag der Anmeldung: **18.06.2008**
(21) Anmeldenummer: 06025798.7
(22) Anmeldetag: 13.12.2006
(51) Int. Cl.: G10L 13/04, G01D 7/12, A61B 5/00

(54) **Sprachausgabegerät und Verfahren zur Sprechtextgenerierung**

(71) Anmelder: F.HOFFMANN-LA ROCHE AG, 4070 Basel (CH); Roche Diagnostics GmbH, 68305 Mannheim (DE)
(72) Erfinder: Kintzig, Hans, 67311 Tiefenthal (DE); Porsch, Ulrich, 69469 Weinheim (DE); Blatt, Christian, 69239 Neckarsteinach (DE)
(74) Vertreter: Hössle, Markus

(57) **Zusammenfassung**

Sprachausgabegerät (10) mit einer ersten Speichereinheit (14), in der Tondateien abgespeichert sind, einer Recheneinheit (12), die einem oder mehreren auszugebenden Datensätzen eine oder mehrere Tondateien aus der ersten Speichereinheit (14) in der richtigen Reihenfolge zuordnet, und einer Ausgabeeinheit (20) mit einer akustischen Datenausgangsschnittstelle (22) zur Wiedergabe der Tondateien in der von der Recheneinheit (12) vorgegebenen Reihenfolge, wobei die Tondateien fixe Tondateien umfassen, die fixe Satzbestandteile enthalten, und variable Tondateien umfassen, die zur Ergänzung der fixen Tondateien dienen, um modulartig vollständige Sätze zu erzeugen.

## Beschreibung

Die vorliegende Erfindung betrifft ein Sprachausgabegerät und ein Verfahren zur Sprechtextgenerierung. Insbesondere betrifft die vorliegende Erfindung ein Sprachausgabegerät zur akustischen Ausgabe von medizinisch relevanten Daten sowie ein Verfahren zur Sprechtextgenerierung von Sätzen und/oder Zahlen zum Betrieb eines derartigen Sprachausgabegeräts.

Im medizinischen Bereich ist es bekannt, tragbare Patientengeräte zum Sammeln von Patientendaten einzusetzen. Häufig sind diese tragbaren Geräte mit zentralen Datenverarbeitungsgeräten verbunden, in denen eine Überwachung, Auswahl, Analyse usw. der Daten entweder von medizinischem Personal, Ärzten oder auch automatisiert vorgenommen wird. Derartige Geräte werden u.a. eingesetzt, um Blutzuckerwerte von Diabetikern zu sammeln und zu überwachen. Aus der EP 1 559 364 A1 ist bspw. ein drahtloses Diabetes-Überwachungssystem bekannt, bei dem dem Patienten nach Übermittlung seiner Blutzuckerwerte an eine Zentrale Verhaltensanweisungen über ein mobiles Telefon mitgeteilt werden. Ein weiteres vergleichbares System ist aus der US 2005/0089150 A1 bekannt, bei dem über Telefon und tragbare Geräte eine interaktive Anweisung eines Nutzers/Patienten mittels Spracherkennungssystemen und softwareerzeugten Anweisungen an den Nutzer erfolgt.

An Diabetes Mellitus erkrankte Personen müssen bestrebt sein, ihren Blutzuckerwert stets in einer bestimmten Höhe zu halten. Wird der angestrebte Bereich überschritten, muss Insulin gespritzt werden. Wird der angestrebte Bereich unterschritten, muss Zucker oral (über die Nahrung oder ein Getränk) zugeführt werden. Wird der angestrebte Bereich über eine längere Zeit überschritten, besteht die Gefahr von ernsthaften gesundheitlichen Komplikationen, wie Blindheit, Nierenschädigung, Absterben von Gliedmaßen oder Neuropathie. Bei kurzfristigem, starkem Überschreiten des Bereiches kann es zu Übelkeit, Schwindel, Schweißausbrüchen und sogar zu zuständen der Verwirrtheit kommen. Bei einem kurzzeitigen Unterschreiten des angestrebten Bereichs kann es ebenfalls zu Übelkeit, Schwindel, Schweißausbrüchen, Verwirrtheit und - im schlimmsten Falle - zum Tode des Diabetikers kommen. Daher ist es dringend geboten, dass ein Diabetiker jederzeit seinen Blutzuckerstatus kennt und ggf. eigenständig geeignete Maßnahmen einleiten kann, um ein Ausbrechen des Blutzuckerwerts aus dem angestrebten Bereich zu vermeiden. Hierzu werden bereits seit geraumer Zeit Blutzuckermessgeräte verwendet, wie sie bspw. aus der DE 10 2004 057 503 A1 bekannt sind und von der Anmelderin unter der eingetragenen Marke Accu-Chek vertrieben werden. Im Idealfall handhabt der Diabetiker die Messung des Blutzukkerwerts und sich daraus ergebende Maßnahmen in Eigenverantwortung.

Der Blutzuckerwert unterliegt starken Schwankungen in Abhängigkeit von den Insulingaben (in der Regel werden unterschiedlich wirkende Insuline gleichzeitig verwendet), von den zugeführten Zuckermengen und anderen physiologisch auf den Zuckerstoffwechsel wirkenden Nahrungs- und Genussmitteln. Ebenfalls auf den Zuckerstoffwechsel wirken körperliche Bewegung, Stress, Krankheit u.v.a.m. Da nicht jeder Organismus in gleicher Weise auf diese physiologischen Größen reagiert, muss jeder Diabetiker seine eigenen physiologischen Reaktionen kennenlernen. Hierzu ist das Führen eines Diabetes-Tagebuchs unerlässlich. Anhand der Eintragungen in ein solches schriftlich geführtes Tagebuch kann der Diabetiker in der Historie seiner Eintragungen ähnliche Situationen suchen und mit der aktuellen Situation vergleichen, um dann entsprechende Maßnahmen zur Korrektur des Stoffwechsels einzuleiten. Er ist aufgrund der Aufzeichnungen in der Lage, erfolgreiche Korrekturen des Stoffwechsels zu wiederholen oder durch entsprechende Anpassungen der Stellglieder, die physiologischen Situationen besser zu korrigieren, als in der Vergangenheit, wenn in einer ähnlichen Situation eine Korrektur nicht zum gewünschten Erfolg geführt hat. Daraus ergibt sich, wie bereits angeführt, eine dringende Notwendigkeit für jeden Diabetiker, ein derartiges Tagebuch zu führen, in dem alle Parameter bzw. Stellglieder des Stoffwechsel-Regelkreises vermerkt werden.

Eine große Anzahl von Diabetikern ist durch Blindheit geschlagen. Ca. 80 % aller blinden Diabetiker haben ihre Blindheit durch ihre Erkrankung, d.h. der Blutzucker dieser Menschen war über eine längere Zeit nicht richtig eingestellt, was zur Erblindung führte. Aufgrund ihrer Erblindung ist es diesen Diabetikern verwehrt, selbst ein Tagebuch wie voranstehend beschrieben zu führen, und sie waren bisher nicht in der Lage, eigenständig eine Insulintherapie durchzuführen. Zwar ist eine Betreuung durch andere Personen möglich, jedoch zeigen Erfahrungswerte, dass dann die Blutzuckereinstellung des Patienten schlechter ist als bei einer eigenverantwortlichen Einstellung des Blutzuckers, d.h. mit einer eigenverantwortlichen Einstellung des Blutzuckers verringert sich die Gefahr von weiteren gesundheitlichen Komplikationen.

Die Gruppe der blinden Diabetiker hat somit ein großes Interesse daran, selbst ein Tagebuch führen und die darin festgehaltene Historie in Form von Daten selektieren zu können, um in kritischen Situationen geeignete Maßnahmen einzuleiten. Mit der parallelen Patentanmeldung EP ... wird ein Sprachausgabegerät vorgeschlagen, das blinden Diabetikern eine derartige Handhabung ihrer Tagebuchdaten ermöglicht.

Im Rahmen der Generierung der Sprechtexte, die von dem Sprachausgabegerät ausgegeben werden sollen, müssen sowohl Wörter und Satzphrasen als auch Zahlen erzeugt werden. Dies erfolgt ausgehend von den Dateien, in denen die auszugebenden Datensätze in dem Sprachausgabegerät vorliegen. Grundsätzlich können zwei Arten der Ausgabe unterschieden werden, nämlich zum einen die Sprachsynthese, bei der Sprachelemente synthetisch gebildet werden, und zum anderen die bloße Wiedergabe von Sprachdatei mit aufgenommenen ("echten") Sprachmustern.

Grundsätzlich sind Verfahren zur Sprachgenerierung bekannt. So ist aus dem US-Patent 4 727 310 ein digitales Volt-Ohm-Meter mit einer Sprachausgabe der gemessenen Werte bekannt. Das bekannte Gerät umfasst einen Sprachsynthesizerschaltkreis, der Sprechtext erzeugt, der aus separaten feststehenden Satzfragmenten kombiniert mit variierenden Bestandteilen (wie bspw. Zahlen) und Einheiten (Volt, Ampere, Ohm) generiert wird und über einen Lautsprecher ausgibt. Zur Generierung des Sprechtextes wird auf abgespeicherte Binärcodes zugegriffen, die jeweils für ein (ebenfalls abgespeicherte) und durch den Synthesizer auszugebende Satzfragment oder eine Einheit stehen. Die Zahlwörter werden direkt anhand der Messpulse des Messgeräts erzeugt, indem entsprechende abgespeicherte Zahlwörter den Einser-, Zehner-, Hunderter- usw. -Werten des Messsignals zugeordnet werden.

Bei der Sprachgenerierung sind grundsätzlich mehrere unterschiedliche Anforderungen zu berücksichtigen. Einerseits sollte der Sprechtext möglichst natürlich und kontinuierlich (also nicht abgehackt) klingen. Insbesondere sollten Zahlen in der gewohnten Sprechweise ausgegeben werden (also "165" als "Hundertfünfundsechzig" und nicht als "eins sechs fünf". Andererseits sollte ein möglichst einfacher Algorithmus zur Spracherzeugung verwendet werden, um die Rechenzeit bzw. den Rechenaufwand zu minimieren. Außerdem soll der benötigte Speicherplatz möglichst klein sein.

Aus dem US-Patent 4 338 490 ist eine synthetische Sprachgenerierung bekannt, bei der Zahlenausgaben unter Verwendung einer vorbestimmten Anzahl von abgespeicherten Phonemen erzeugt werden, wobei ein komplexer Algorithmus zur Bestimmung der Fließkommaposition und von eventuell in die Sprachausgabe einzufügenden Pausen vorgesehen ist.

Aus dem US-Patent 4 707 794 ist ein synthetischer Sprachgenerator mit einem Rechenschaltkreis mit mehreren Speicherplätzen bekannt, der als Sprachtaschenrechner dient. Für die Sprachgenerierung ist ein umfangreiches Lexikon vorgesehen, mit dem komplexe Rechengleichungen von Textdaten in gesprochene Daten umgeformt werden können, womit ein sehr hoher Speicherbedarf verbunden ist.

Erfindungsgemäß wird daher vorgeschlagen, eine Sprechtexterzeugung auf der Grundlage von abgespeicherten und modulartig kombinierbaren Tondateien durchzuführen. Die Tondateien umfassen zum einen sogenannte fixe Tondateien, die fixe Satzphrasen bzw. Satzbestandteile enthalten. Zum anderen umfassen die Tondateien sogenannte variable Tondateien, die zur Ergänzung der fixen Tondateien dienen.

Somit können erfindungsgemäß auf eine einfache, aber effektive Art und Weise und mit nur wenig Speicherplatzbedarf eine Vielzahl von bei einem Sprachausgabegerät auftretenden Sprachkonstellationen bzw. Satzkonstellationen erzeugt werden. Die fixen Tondateien können dazu Variablenpositionen aufweisen, in die die variablen Tondateien "eingesetzt" werden können. Diese Variablenpositionen können am Anfang und/oder am Ende und/oder an einer anderen Stelle innerhalb der fixen Tondatei liegen. Je nach Variablenposition wird die einzusetzende variable Tondatei der fixen Tondatei vorangestellt oder nachgestellt oder in sie eingesetzt. Letzteres kann dadurch erfolgen, dass die Wiedergabe der fixen Tondatei an der Stelle der Variablenposition unterbrochen wird, solange die einzusetzende variable Tondatei wiedergegeben wird. Selbstverständlich kann eine fixe Tondatei mehr als eine Variablenposition umfassen.

Zahlen können im Rahmen der Erfindung aus mehreren variablen Tondateien zusammengesetzt werden. Dazu sind bspw. zur Erzeugung deutschsprachiger Zahlbegriffe eine variable Tondatei für jede Zahl von Null bis 99 vorgesehen und jeweils eine variable Tondatei für jeden Hunderter, Tausender usw. vorgesehen. Aus diesen variablen Tondateien kann jede beliebige Zahl zusammengesetzt werden, ohne dass die Sprachmelodie bzw. der natürliche Sprachfluss leidet. Des weiteren können geeignete Zusatzdateien vorgesehen sein, wie bspw. "und" im Deutschen.

Die Erfindung umfasst auch ein Computerprogramm mit Programmcode, der dazu geeignet ist, ein wie Verfahren gemäß der Erfindung durchzuführen, wenn das Computerprogramm auf einem geeigneten Rechengerät, bspw. einem Sprachausgabegerät mit einer Recheneinheit, abläuft. Das Computerprogramm kann als sogenannte Embedded Software auf einem Sprachausgabegerät gespeichert sein, es kann aber auch über eine geeignete Schnittstelle von einem geeigneten Medium auf das Sprachausgabegerät überspielt werden.

Weitere Vorteile und Ausgestaltungen der Erfindung ergeben sich aus der Beschreibung und der beiliegenden Zeichnung.

Es versteht sich, dass die voranstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung ist anhand eines Ausführungsbeispieles in der Zeichnung schematisch dargestellt und wird im folgenden unter Bezugnahme auf die Zeichnung ausführlich beschrieben.

Figur 1 zeigt in schematischer perspektivischer Ansicht ein Ausführungsbeispiel eines erfindungsgemäßen Sprachausgabegeräts.

Figur 2 zeigt ein den Aufbau des Sprachausgabegeräts der Figur 1 darstellendes Blockdiagramm.

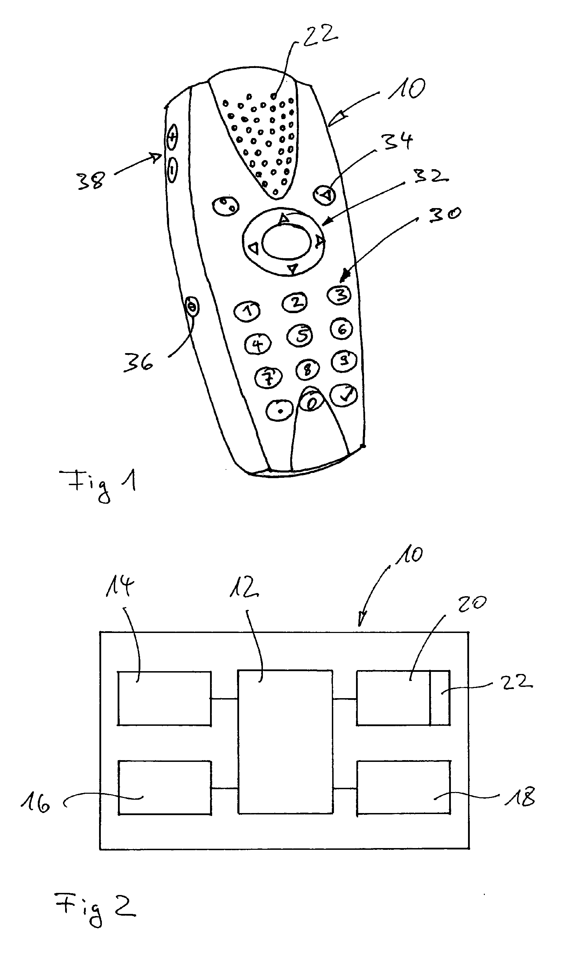

Ein erfindungsgemäßes Sprachausgabegerät 10 ist in Figur 1 in perspektivischer Darstellung und in schematischer Blockdarstellung in Figur 2 gezeigt.

Das Sprachausgabegerät 10 umfasst eine Recheneinheit 12, eine erste Speichereinheit 14, eine zweite Speichereinheit 16, eine Eingabeeinheit 18 sowie eine Ausgabeeinheit 20 mit einer akustischen Datenausgangsschnittstelle 22. Die akustische Datenausgangsschnittstelle 22 ist bspw. ein Lautsprecher (vgl. Figur 1) und/oder eine Kopf- bzw. Ohrhörerbuchse.

Des weiteren umfasst das Sprachausgabegerät 10 eine Mehrzahl von (nicht näher bezeichneten und Bestandteil der Eingabeeinheit 18 bildenden) Tasten, anhand derer eine Bedienperson das Sprachausgabegerät 10 bedienen und benutzen kann. Bei den Tasten handelt es sich insbesondere um eine numerische Tastatur 30 (im gezeigten Ausführungsbeispiel wie bei einem Telefon angeordnet) sowie um Steuerungstasten 32 (Pfeiltasten), Eingabebestätigungstaste 34, Ein/Aus-Taste 36, +/- Tasten 38 zur Lautstärkeeinstellung u.a. Es versteht sich, dass die Ausgestaltung der Eingabeeinheit und insbesondere Art und Weise und Umfang der Tastatur nicht auf die dargestellte Ausführungsform beschränkt ist und der Fachmann auch anders gestaltete Tastaturanordnungen in Betracht zieht.

Die Eingabeeinheit umfasst des weiteren (nicht dargestellte) Schnittstellen zur Dateneingabe, wie bspw. eine Infrarotschnittstelle, eine serielle Datenschnittstelle und/oder eine USB-Schnittstelle. Alternativ kann bspw. auch eine Bluetooth-Schnittstelle o.dgl. vorgesehen sein.

In der ersten Speichereinheit 14 sind erfindungsgemäß Tondateien abgespeichert, aus denen sich die Sprachausgabe des Sprachausgabegeräts erzeugen lässt. Die Tondateien umfassen fixe Tondateien und variable Tondateien. Unter fixen Tondateien sind im Kontext dieser Anmeldung Tondateien zu verstehen, die einen fixen Satzbestandteil oder mit anderen Worten einen festen, unveränderlichen Rumpf- oder Basissatz enthalten. Dabei handelt es sich um einen vollständigen oder fast vollständigen Satz, der in einer gegebenen Situation von dem Sprachausgabegerät 10 auszugeben ist. Handelt es sich um einen vollständigen Satz, so wird die entsprechende Tondatei im gegebenen Fall einfach über die Ausgabeeinheit 20 wiedergegeben. Handelt es sich um einen nichtvollständigen Satz, so muss dieser vor der Wiedergabe ergänzt werden. Diese Ergänzung erfolgt erfindungsgemäß anhand einer variablen Tondatei. Unter einer variablen Tondatei ist im Kontext dieser Anmeldung eine Tondatei zu verstehen, die einzelne Worte oder kurze Satzfragmente enthält, die modulartig mit einer oder mehreren anderen variablen Tondateien und/oder einer oder mehreren fixen Tondateien kombinierbar ist.

Beispielsweise könnte in einer gegebenen Situation eine Sprachausgabe erfolgen, die den Benutzer darauf hinweist dass er eine falsche Taste gedrückt hat oder eine bestimmte Taste nicht richtig gedrückt hat. Angenommen, der Benutzer hat die Bestätigungstaste (in der Darstellung der Figur 1 untern rechts) nicht korrekt gedrückt. In einem solchen Fall soll die Sprachausgabe lauten: "Sie haben die Bestätigungstaste nicht korrekt gedrückt." Um zu vermeiden, dass für jede mögliche Taste eine fixe Tondatei mit einem vollständigen Satz besprochen und abgespeichert werden muss, wird erfindungsgemäß lediglich der Rumpfsatz "Sie haben die ... nicht korrekt gedrückt." abgespeichert. Der (weggelassene) Satzbestandteil "Bestätigungstaste" wird als variable Tondatei hinterlegt.

In dem beschriebenen Beispiel stellt die Satzlücke zwischen den beiden Satzteilen "Sie haben die" und "nicht korrekt gedrückt" die sogenannte Variablenposition dar. Dieser Variablenposition wird die entsprechende variable Tondatei "Bestätigungstaste" von der Recheneinheit zugeordnet, wenn der beschriebene Satz "Sie haben die Bestätigungstaste nicht korrekt gedrückt." auszugeben ist. Es handelt sich bei der fixen Tondatei dieses Beispiels somit um eine fixe Tondatei mit genau einer Variablenposition. Für die Bestätigungstaste und jede weitere Taste gibt es jeweils eine variable Tondatei, so dass mit diesem Satz an Tondateien eine passende Sprachausgabe bei nicht korrektem Drücken jeder beliebigen Taste erzeugt werden kann.

Eine weitere fixe Tondatei könnte bspw. lauten: "Bitte drücken Sie die ...". Bei dieser fixen Tondatei liegt die Variablenposition am Ende und kann durch eine der bereits vorhandenen variablen Tondateien mit den Tastennamen ergänzt werden.

Bei der Ausgabe bzw. Wiedergabe der fixen Tondatei wird die entsprechende variable Tondatei der fixen Tondatei je nach Stelle der Variablenposition einfach voran- oder nachgestellt. In dem letzteren Beispiel würde die variable Tondatei nachgestellt werden. Soll der Benutzer bspw. aufgefordert werden, die Bestätigungstaste zu drücken, so würde zunächst die fixe Tondatei "Bitte drücken Sie die ..." abgespielt werden, direkt gefolgt von der variablen Tondatei "Bestätigungstaste". In dem zuerst beschriebenen Beispiel, in dem sich die Variablenposition innerhalb der fixen Tondatei befindet, wird die Wiedergabe der fixen Tondatei "Sie haben die ... nicht korrekt gedrückt." bei Erreichen der Variablenposition unterbrochen bzw. gestoppt und es wird die einzufügende variable Tondatei "Bestätigungstaste" abgespielt, um anschließend mit der Wiedergabe der fixen Tondatei fortzufahren.

Auf die gleiche Art und Weise lässt sich erfindungsgemäß eine Erzeugung von Zahlwörtern anhand modulartig kombinierbarer variabler Tondateien durchführen. Für die Erzeugung deutschsprachiger Zahlwörter wird hierzu ein Satz variabler Tondateien abgespeichert, die jeweils eine der Zahlen Null bis 99 in gesprochener Sprache enthalten. Zur Erzeugung höherer Zahlen werden für jeden Hunderter (100, 200, 300, ..., 900), jeden Tausender (1000, 2000, 3000, ..., 9000) usw. eine variable Tondatei angelegt. Um den Zahlenwert sprachlich exakt darzustellen, können noch geeignete Zusatzdateien, wie bspw. "und", abgelegt werden.

So können für Fälle, in denen maximal vierstellige Zahlenwerte sprachlich ausgegeben werden müssen, mit lediglich 118 Dateien sämtliche Zahlenwerte zwischen Null und 9999 erzeugt werden, ohne dass komplexe Algorithmen oder Spracherzeugungsmodule notwendig wären. Gleichzeitig wird nur wenig Speicherplatz benötigt, da es sich um durchweg kurze Sprach- bzw. Tondateien handelt. Auch Jahreszahlen können aus diesen Dateien erzeugt werden. So kann die Zahl 1963 sowohl als Kombination der Dateien "Tausend", "Neunhundert", "Dreiundsechzig" als auch als Kombination der Dateien "Neunzehn", "Hundert", "Dreiundsechzig" erzeugt werden.

Die notwendigen und ggf. je nach Konstellation oder Zusammenhang (wie bspw. bei Jahreszahlen) angebrachten Kombinationen werden von der Recheneinheit anhand von hinterlegten Matrizen, die ebenfalls wenig Speicherplatz benötigen, errechnet. Da es sich um einfache Berechnungen handelt, wird auch keine hohe Rechenkapazität benötigt.

Auszugebende Daten bzw. Datensätze können über die Eingabeeinheit direkt eingegeben werden oder in der zweiten Speichereinheit hinterlegt werden. Handelt es sich bei dem Sprachausgabegerät bspw. um ein Sprachausgabegerät im medizinischen Bereich, so werden die Daten als Messdaten erhoben und über eine Schnittstelle (bspw. Infrarotschnittstelle) in die zweite Speichereinheit 16 des Sprachausgabegeräts gespeichert und von dort bei Bedarf von der Recheneinheit aufgerufen und ggf. mit fixen Tondateien aus der ersten Speichereinheit kombiniert. So können bspw. Sprachausgaben wie "Ihr Insulinwert vom 12. Oktober 2006, 12:08 Uhr, beträgt 104" realisiert werden:
1. fixe Tondatei (für den wiederkehrenden Rumpfsatz): "Ihr Insulinwert vom [Variablenposition Datum] [Variablenposition Uhrzeit] beträgt [Variablenposition Insulinwert]"
2. variable Tondateien für Datum: "Zwölfter" + "Oktober" + "Zweitausend" + "Sechs"
3. variable Tondateien für Uhrzeit: "Zwölf" + "Uhr" + "Acht"
4. variable Tondateien für Insulinwert: "Hundert" + "Vier"

Die vorliegende und voranstehend ausführlich beschriebene Erfindung ermöglicht es, mit einem geringen Aufwand an Rechenkapazität und Speicherplatz eine Sprachausgabe mit natürlicher Satzstellung und Satzmelodie zu erzeugen. Damit wird die Verständlichkeit der Sprachausgabe deutlich verbessert, was insbesondere im Bereich der Sprachausgabe medizinischer Daten äußerst wichtig ist, um die Gefahr von Missverständnissen und Fehlinterpretationen auszuschließen bzw. zu verringern. Erfindungsgemäß wird der Speicherbedarf durch geschickte Kombination von Phrasen (Rumpfsätzen) und Einsetzen von Variablen an entsprechenden Stellen so weit wie möglich reduziert, ohne dass daraus Abstriche in Satzmelodie und entsprechenden grammatikalischen Eigenheiten (auch in verschiedenen Sprachen) folgen.

## Patentansprüche

1. Sprachausgabegerät (10) mit einer ersten Speichereinheit (14), in der Tondateien abgespeichert sind, einer Recheneinheit (12), die einem oder mehreren auszugebenden Datensätzen eine oder mehrere Tondateien aus der ersten Speichereinheit (14) in der richtigen Reihenfolge zuordnet, und einer Ausgabeeinheit (20) mit einer akustischen Datenausgangsschnittstelle (22) zur Wiedergabe der Tondateien in der von der Recheneinheit (12) vorgegebenen Reihenfolge, wobei die Tondateien fixe Tondateien umfassen, die fixe Satzbestandteile enthalten, und variable Tondateien umfassen, die zur Ergänzung der fixen Tondateien dienen, um modulartig vollständige Sätze zu erzeugen.

2. Sprachausgabegerät mit einer ersten Speichereinheit, in der Tondateien abgespeichert sind, einer Recheneinheit, die einem oder mehreren auszugebenden Datensätzen eine oder mehrere Tondateien aus der ersten Speichereinheit in der richtigen Reihenfolge zuordnet, und einer Ausgabeeinheit mit einer akustischen Datenausgangsschnittstelle zur Wiedergabe der Tondateien in der von der Recheneinheit vorgegebenen Reihenfolge, wobei die Tondateien variable Tondateien umfassen, die modulartig kombinierbar sind, um Zahlwörter zu erzeugen.

3. Sprachausgabegerät nach Anspruch 2, bei dem die Tondateien des weiteren fixe Tondateien zur Kombination mit den variablen Tondateien umfassen.

4. Sprachausgabegerät nach Anspruch 1 oder 3, bei dem jede fixe Tondatei mindestens eine Variablenposition umfasst, die bei der Wiedergabe durch eine variable Tondatei ergänzt wird.

5. Sprachausgabegerät nach Anspruch 4, bei dem die Variablenposition der fixen Tondatei am Anfang und/oder am Ende und/oder an einer Stelle innerhalb der fixen Tondatei ist.

6. Sprachausgabegerät nach Anspruch 5, bei dem bei der Wiedergabe entsprechend der Variablenposition ein Voranstellen bzw. ein Nachstellen der variablen Tondatei vor bzw. nach der fixen Tondatei bzw. ein Einsetzen der variablen Tondatei in die fixe Tondatei erfolgt.

7. Sprachausgabegerät nach Anspruch 4 oder 6, bei dem eine Unterbrechung der Wiedergabe der fixen Tondatei an der Variablenposition erfolgt, solange eine Wiedergabe der variablen Tondatei erfolgt.

8. Sprachausgabegerät nach einem der Ansprüche 2 bis 7, bei dem die variablen Tondateien derart aufgebaut sind, dass eine variable Tondatei für jede Zahl von Null bis 99 vorgesehen ist und dass für jeden Hunderter, Tausender usw. jeweils eine variable Tondatei vorgesehen ist.

9. Verfahren zur Sprechtextgenerierung für ein Sprachausgabegerät basierend auf einem Satz von Tondateien, wobei der Satz von Tondateien fixe Tondateien umfasst, die fixe Satzbestandteile enthalten, und variable Tondateien umfasst, die zur Ergänzung der fixen Tondateien dienen, um modulartig vollständige Sätze zu erzeugen.

10. Verfahren zur Sprechtextgenerierung für ein Sprachausgabegerät basierend auf einem Satz von Tondateien, wobei der Satz von Tondateien variable Tondateien umfasst, die modulartig kombinierbar sind, um Zahlwörter zu erzeugen.

11. Verfahren nach Anspruch 10, bei dem zusätzlich fixe Tondateien vorgesehen sind, die mit den variablen Tondateien zur modulartigen Erzeugung von vollständigen Sätzen kombinierbar sind.

12. Verfahren nach Anspruch 9 oder 11, bei dem jede fixe Tondatei mindestens eine Variablenposition umfasst, die bei der Wiedergabe durch eine variable Tondatei ergänzt wird.

13. Verfahren nach Anspruch 12, bei dem die Variablenposition der fixen Tondatei am Anfang und/oder am Ende und/oder an einer Stelle innerhalb der fixen Tondatei ist.

14. Verfahren nach Anspruch 13, bei dem die variable Tondatei bei der Wiedergabe entsprechend der Variablenposition der fixen Tondatei vorangestellt bzw. nachgestellt bzw. in die fixe Tondatei eingesetzt wird.

15. Verfahren nach Anspruch 12 oder 14, bei dem eine Wiedergabe der fixen Tondatei an der Variablenposition unterbrochen wird, solange die variable Tondatei wiedergegeben wird.

16. Verfahren nach Anspruch 9, bei dem eine Tondatei Wörter bzw. Satzphrasen und/oder Ziffern bzw. Zahlen umfassen kann.

17. Verfahren nach Anspruch 10, bei dem die variablen Tondateien derart aufgebaut sind, dass eine variable Tondatei für jede Zahl von Null bis 99 vorgesehen ist und dass für jeden Hunderter, Tausender usw. jeweils eine variable Tondatei vorgesehen ist.

18. Computerprogramm mit Programmcode, der dazu geeignet ist, ein Verfahren nach einem der Ansprüche 9 bis 17 auszuführen, wenn das Computerprogramm auf einer Recheneinrichtung abläuft.

19. Computerprogramm nach Anspruch 18, das auf einem computerlesbaren Medium gespeichert ist.
